(19) Europäisches Patentamt
European Patent Office
Office européen des brevets

(11) Numéro de publication : **0 521 781 A1**

(12) # DEMANDE DE BREVET EUROPEEN

(21) Numéro de dépôt : **92401873.2**

(51) Int. Cl.$^5$ : **C07D 277/34,** A01N 53/00

(22) Date de dépôt : **01.07.92**

(30) Priorité : **04.07.91 FR 9108380**

(43) Date de publication de la demande : **07.01.93 Bulletin 93/01**

(84) Etats contractants désignés : **CH DE FR GB IT LI NL**

(71) Demandeur : **ROUSSEL-UCLAF**
**35, Boulevard des Invalides**
**F-75007 Paris (FR)**

(72) Inventeur : **Benoit, Marc**
**Le Cannet Est, Pont de l'Etoile**
**F-13360 Roquevaire (FR)**
Inventeur : **Demassey, Jacques**
**Allée Saint Jacques Chalifert**
**F-77144 Montevrain (FR)**
Inventeur : **Demoute, Jean-Pierre**
**65, Avenue Foch**
**F-93360 Neuilly Plaisance (FR)**

(74) Mandataire : **Tonnellier, Marie-José et al**
**111, route de Noisy B.P. no 9**
**F-93230 Romainville (FR)**

(54) **Nouveaux esters pyréthrinoides de l'alcool 2,3-dihydro 4-méthyl 2-oxo 3-(2-propynyl) thiazol 5-yl méthylique, leur procédé de préparation et leur application comme pesticides.**

(57) L'invention a pour objet les composés de formule (I) :

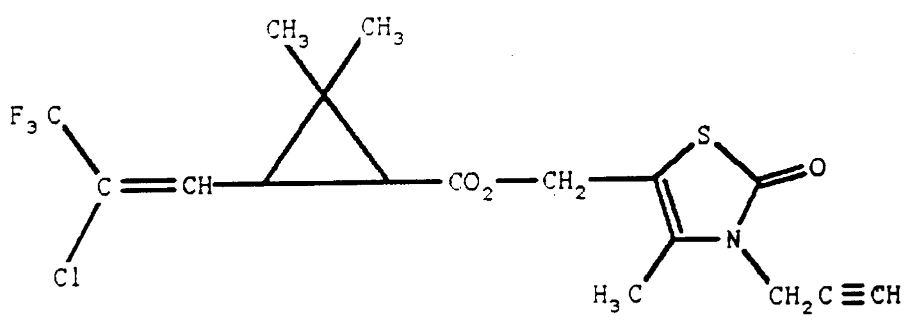

(I)

sous toutes leurs formes stéréoisomères possibles ainsi que leurs mélanges.

Les composés de formule (I) présentent d'intéressantes propriétés pesticides.

EP 0 521 781 A1

La présente invention concerne de nouveaux esters pyréthrinoïdes de l'alcool 2,3-dihydro 4-méthyl 2-oxo 3-(2-propynyl) thiazol 5-yl méthylique, leur procédé de préparation et leur application comme pesticides.

L'invention a pour objet les composés de formule (I) :

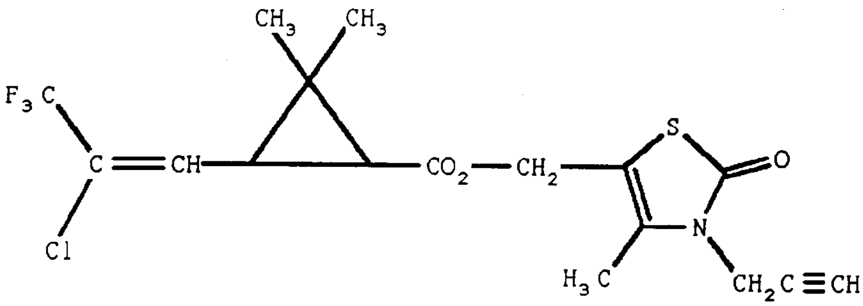

(I)

sous toutes leurs formes stéréoisomères possibles ainsi que leurs mélanges.

L'invention a plus particulièrement pour objet :

- les composés de formule (I) dans lesquels la copule cyclopropanique est de structure 1Rcis,
- les composés de formule (I) dans lesquels la géométrie de la double liaison est Z.

Parmi les composés préférés de l'invention, on peut citer le [1R[1alpha,3alpha(Z)]] 3-[(2-chloro 2-trifluorométhyl) éthényl] 2,2-diméthyl cyclopropane carboxylate de [2,3-dihydro 4-méthyl 2-oxo 3-(2-propynyl) thiazol 5-yl] méthyle.

Les composés de formule (I) présentent d'intéressantes propriétés qui permettent leur utilisation dans la lutte contre les parasites. Il peut s'agir par exemple de la lutte contre les parasites des locaux, les parasites des végétaux et les parasites des animaux à sang chaud. C'est ainsi que l'on peut utiliser les produits de l'invention pour lutter contre les insectes, les nématodes et les acariens parasites des végétaux et des animaux.

L'invention a donc pour objet l'application des composés de formule (I) à la lutte contre les parasites des locaux, les parasites des végétaux et les parasites des animaux à sang chaud.

Les composés de formule (I) peuvent être utilisés pour lutter contre les insectes dans les locaux, pour lutter notamment contre les mouches, les moustiques et les blattes.

Les produits de formule (I) peuvent être utilisés également pour lutter contre les insectes dans le domaine agricole, pour lutter par exemple, contre les pucerons, les larves de lépidoptères et les coléoptères. Ils sont utilisés à des doses comprises entre 10 g et 300 g de matière active à l'hectare.

Les composés de formule (I) présentent notamment un très bon pouvoir de knock down.

Il ressort des résultats de tests biologiques ci-après que les produits de formule (I) possèdent également une remarquable activité aphicide.

Les produits de formule (I) peuvent aussi être utilisés pour lutter contre les acariens parasites des végétaux.

Les composés de formule (I) peuvent aussi être utilisés pour lutter contre les nématodes parasites des végétaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomnia, ceux de l'espèce Amblyomnia et ceux de l'espèce Rhipicephalus, ou pour lutter contre toutes sortes de gales et notamment la gale sarcoptique, la gale psoroptique et la gale chorioptique.

L'invention a donc également pour objet les compositions destinées à la lutte contre les parasites des végétaux, les parasites des locaux et les parasites des animaux à sang chaud, caractérisées en ce qu'elles renferment au moins l'un des produits définis ci-dessus.

Les compositions selon l'invention sont préparées selon les procédés usuels de l'industrie agrochimique ou de l'industrie vétérinaire ou de l'industrie des produits destinés à la nutrition animale.

Ces compositions peuvent se présenter sous forme de poudres, granulés, suspensions, émulsions, solutions, solutions pour aérosols, bandes combustibles, appâts ou autres préparations employées classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre telle que le talc, les argiles, les silicates, le kieselguhr ou un solide combustible.

L'invention a notamment pour objet les compositions insecticides renfermant comme principe actif au

moins l'un des produits définis ci-dessus, et plus particulièrement les compositions renfermant comme principe actif le produit de l'exemple 1.

Les compositions insecticides selon l'invention contiennent de préférence de 0,005 % à 10 % en poids de matière active.

Selon un mode opératoire avantageux, pour un usage dans les locaux, les compositions de l'invention peuvent se présenter sous forme d'appâts. Les compositions insecticides selon l'invention peuvent également être utilisées sous forme de compositions fumigantes.

Les compositions selon l'invention peuvent alors être avantageusement constituées, pour la partie non active, d'un serpentin insecticide (ou coil) combustible, ou encore d'un substrat fibreux incombustible. Dans ce dernier cas, le fumigant obtenu après incorporation de la matière active est placé sur un appareil chauffant tel qu'un émanateur électrique.

Dans le cas où l'on utilise un serpentin insecticide, le support inerte peut être, par exemple, composé de marc de pyrèthre, poudre de Tabu (ou poudre de feuilles Machilus Thumbergii), poudre de tige de pyrèthre, poudre de feuille de cèdre, poudre de bois (telle que de la sciure de pin) amidon et poudre de coque de noix de coco. La dose de matière active peut alors être, par exemple, de 0,03 à 1 % en poids.

Dans le cas où l'on utilise un support fibreux incombustible, la dose de matière active peut alors être, par exemple, de 0,03 à 95 % en poids.

Les compositions selon l'invention pour un usage dans les locaux peuvent aussi être obtenues en préparant une huile pulvérisable à base de principe actif, cette huile imbibant la mèche d'une lampe et étant alors soumise à la combustion.

La concentration du principe actif incorporé à l'huile est, de préférence, de 0,03 à 95 % en poids.

L'invention a également pour objet, les compositions acaricides renfermant comme principe actif au moins un des produits de formule (I) définis ci-dessus.

L'invention a également pour objet les compositions nématicides renfermant comme principe actif au moins un des produits de formule (I) ci-dessus.

Les compositions insecticides selon l'invention, comme les compositions acaricides et nématicides, peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables, pour pulvérisation foliaire, contenant de 1 à 80 % ou des liquides pour pulvérisation foliaire contenant de 1 à 500 g/l de principe actif. On peut également employer des poudres pour poudrage foliaire contenant de 0,05 à 3 % de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

L'invention a également pour objet les compositions acaricides destinées à la lutte contre les acariens parasites des animaux à sang chaud, notamment contre les tiques et les gales, caractérisées en ce qu'elles renferment comme principe actif, au moins l'un des produits de formule (I) définie ci-dessus.

Lorsqu'il s'agit de lutter contre les acariens parasites des animaux, on peut incorporer les produits de l'invention dans des compositions alimentaires en association avec un mélange nutritif adapté à l'alimentation animale. Le mélange nutritiel peut varier selon l'espèce animale, il peut renfermer des céréales, des sucres et des grains, des tourteaux de soja, d'arachide et de tournesol, des farines d'origine animale, par exemple des farines de poissons, des acides aminés de synthèse, des sels minéraux, des vitamines et des antioxydants.

L'invention a donc également pour objet les compositions destinées à l'alimentation animale, renfermant comme principe actif au moins l'un des produits de formule (I) telle que définie précédemment.

On peut indiquer également que les produits de l'invention peuvent être utilisés comme biocides ou comme régulateur de croissance.

L'invention a également pour objet les associations douées d'activité insecticide, acaricide ou nématicide, caractérisées en ce qu'elles contiennent comme matière active, d'une part un au moins des composés de formule générale (I), et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3,4,5,6-tétrahydrothiophénylidène méthyl) cyclopropanecarboxyliques, par les esters d'alcool 3-phénoxy benzyliques et d'alcools alpha-cyano 3-phénoxy benzyliques des acides 2,2-diméthyl 3-(2,2-dichlorovinyl) cyclopropanecarboxyliques, par les esters d'alcools alpha-cyano 3-phénoxy benzylique d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides 2,2-diméthyl 3-(1,2,2,2-tétrahalo éthyl) cyclopropanecarboxyliques, dans lesquels "halo" représente un atome de fluor, de chlore ou de brome, étant entendu que les composés (I) peuvent exister sous toutes leurs formes stéréoisomères possibles de même que les copules acides et alcools des esters pyréthrinoïdes ci-dessus.

Les associations selon l'invention présentent notamment l'intérêt soit de permettre de combattre, par la polyvalence de leur action, une gamme de parasites plus étendue, soit de manifester, dans certains cas, un effet de synergie.

Pour exalter l'activité biologique des produits de l'invention, on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo[2,2-1]5-heptène-2,3-dicarboximide, ou le pipéronyl-bis-2-(2'-n-butoxy éthoxy) éthylacétal (ou tropital).

L'invention a donc pour objet les compositions pesticides définies précédemment, caractérisées en ce qu'elles renferment en outre un synergiste des pyréthrinoïdes.

L'invention a également pour objet un procédé de préparation, caractérisé en ce que l'on soumet un acide de formule (II) :

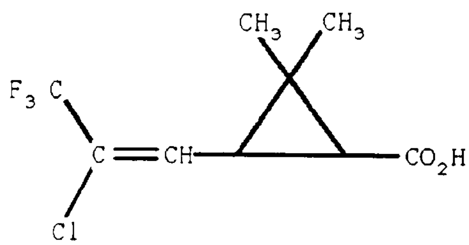

(II)

ou un dérivé fonctionnel de cet acide, avec un alcool de formule (III) :

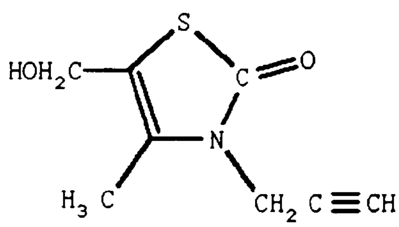

(III)

ou un dérivé fonctionnel de cet alcool, pour obtenir le composé de formule (I) correspondant.

Le dérivé fonctionnel d'acide utilisé est de préférence un chlorure d'acide.

Lorsque l'on fait réagir l'acide de formule (II) et l'alcool de formule (III), on opère de préférence en présence de dicyclohexyl carbodiimide.

L'alcool de formule (III) est décrit dans le brevet européen n° 0300898.

Les exemples suivants illustrent l'invention sans toutefois la limiter.

**EXEMPLE 1 : [1R[1alpha,3alpha(Z)] 3-[2-(chloro 2-trifluorométhyl) éthényl] 2,2-diméthyl cyclopropanecarboxylate de [2,3-dihydro 4-méthyl 2-oxo 3-(2-propynyl) thiazol 5-yl] méthyle**

On introduit à 0/5°C, un mélange renfermant 1,70 g de dicyclohexylcarbodiimide, 100 mg de 4-diméthylaminopyridine et 20 cm³ de chlorure de méthylène, dans un mélange de 2 g d'acide [1R[1alpha,3alpha(Z)] 3-[2-(chloro 2-trifluorométhyl) éthényl] 2,2-diméthyl cyclopropanecarboxylique et 1,40 g d'alcool 2,3-dihydro 4-méthyl 3-(2-propynyl) thiazol 5-yl méthylique et 70 cm³ de chlorure de méthylène. On agite pendant un quart d'heure à 0°C, puis laisse revenir à 20°C et agite pendant 2 heures. On filtre et concentre sous pression réduite. On obtient 3,6 g d'un produit que l'on chromatographie sur silice, en éluant avec le mélange cyclohexane, acétate d'éthyle 8-2. On évapore le solvant, on sèche et obtient 2,75 g d'un produit que l'on chromatographie sur silice avec le mélange hexane-acétate d'éthyle (7-3). On évapore et sèche. On obtient ainsi 2,35 g du produit recherché.

$alpha_D$ = +27°5 ± 1,5° (c = 0,7 % $CHCl_3$)

**EXEMPLE : Préparation d'une composition fumigène**

On mélange d'une façon homogène :

| | |
|---|---|
| produit de l'exemple 3 | 0,25 g |
| poudre de tabu | 25,00 g |
| poudre de feuille de cèdre | 40,00 g |
| poudre de bois de pin | 33,75 g |
| vert brillant | 0,50 g |
| p-Nitrophénol | 0,50 g |

**ETUDE BIOLOGIQUE**

A - Etude de l'effet d'abattage sur mouche domestique

Les insectes tests sont des mouches domestiques femelles âgées de 4 jours. On opère par pulvérisation directe à la concentration de 0,10 g/l en chambre de Kearns et March en utilisant comme solvant un mélange d'acétone (5 %) et d'Isopar L (solvant pétrolier) (quantité de solvant utilisée 2 $cm^3$ en une seconde). On utilise 50 insectes par traitement. On effectue les contrôles toutes les minutes jusqu'à 10 minutes, puis à 15 minutes et l'on détermine le $KT_{50}$ par les méthodes habituelles.

Les résultats expérimentaux obtenus sont résumés dans le tableau suivant, en puissance relative par rapport à la bioalléthrine.

| COMPOSES | PR |
|---|---|
| EXEMPLE 1 | 6,65 |

**Revendications**

**1)** Les composés de formule (I) :

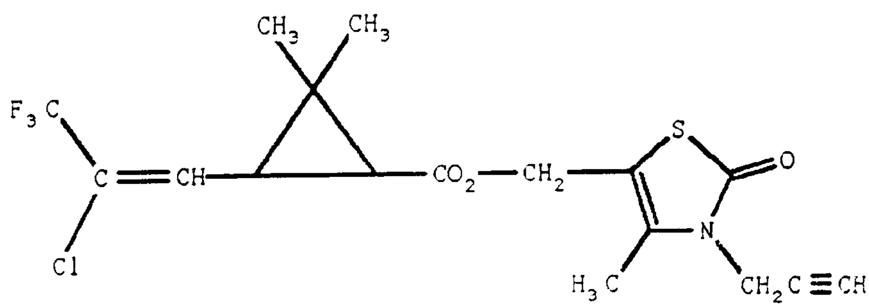

(I)

sous toutes leurs formes stéréoisomères possibles ainsi que leurs mélanges.

**2)** Les composés de formule (I) tels que définis à la revendication 1 dans lesquels la copule cyclopropanique est de structure 1Rcis.

**3)** Les composés de formule (I) tels que définis à la revendication 1 ou 2 dans lesquels la géométrie de la double liaison est Z.

**4)** Le [1R[1alpha,3alpha(Z)]] 3-[(2-chloro 2-trifluorométhyl) éthényl] 2,2-diméthyl cyclopropanecarboxylate de [2,3-dihydro 4-méthyl 2-oxo 3-(2-propynyl) thiazol 5-yl] méthyle.

**5)** Application des composés de formule (I) tels que définis à l'une quelconque des revendications 1 à 4 à la lutte contre les parasites des locaux, les parasites des végétaux et les parasites des animaux à sang chaud.

**6)** Les compositions destinées à la lutte contre les parasites des locaux, les parasites des végétaux et les parasites des animaux à sang chaud, caractérisées en ce qu'elles renferment comme principe actif au moins un des produits définis à l'une quelconque des revendications 1 à 3.

**7)** Les compositions destinées à la lutte contre les parasites des locaux, les parasites des végétaux et les parasites des animaux à sang chaud, caractérisées en ce qu'elles renferment comme principe actif au moins un des produits définis à la revendication 4.

**8)** Les compositions insecticides renfermant comme principe actif au moins l'un des produits définis à l'une quelconque des revendications 1 à 4.

**9)** Les compositions insecticides selon la revendication 8 se présentent sous forme d'appâts.

**10)** Les compositions acaricides renfermant comme principe actif au moins l'un des produits définis à l'une quelconque des revendications 1 à 4.

**11)** Les compositions nématicides renfermant comme principe actif au moins l'un des produits définis à l'une quelconque des revendications 1 à 4.

**12)** Les compositions acaricides destinées à la lutte contre les acariens parasites des animaux à sang chaud, notamment contre les tiques et les gales, caractérisées en ce qu'elles renferment comme principe actif, au moins l'un des produits définis à l'une quelconque des revendications 1 à 4.

**13)** Associations douées d'activité insecticide, acaricide ou nématicide, caractérisées en ce qu'elles contiennent comme matière active, d'une part un au moins des composés de formule générale (I) telle que définie à la revendication 1, et d'autre part, un au moins des esters pyréthrinoïdes choisis dans le groupe constitué par les esters d'alléthrolone, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxy benzyliques des acides chrysanthémiques, par les esters d'alcool 5-benzyl 3-furyl méthylique des acides 2,2-diméthyl 3-(2-oxo 3-tétrahydrothiophénylidène méthyl) cyclopropanecarboxyliques, par les esters d'alcool 3-phénoxy benzylique et d'alcool alpha-cyano 3-phénoxy benzyliques des acides 2,2-diméthyl 3-(2,2-dichlorovinyl ) cyclopropanecarboxyliques, par les esters d'alcools alpha-cyano 3-phénoxy benzyliques d'acides 2,2-diméthyl 3-(2,2-dibromovinyl) cyclopropanecarboxyliques, par les esters d'alcool 3-phénoxy benzylique des acides 2-parachlorophényl 2-isopropyl acétiques, par les esters d'alléthrolone, d'alcool 3,4,5,6-tétrahydrophtalimido méthylique, d'alcool 5-benzyl 3-furyl méthylique, d'alcool 3-phénoxy benzylique et d'alcools alpha-cyano 3-phénoxybenzyliques des acides 2,2-diméthyl 3-(1,2,2,2-tétrahaloéthyl) cyclopropanecarboxyliques, dans lesquels "halo" représente un atome de fluor, de chlore ou de brome, étant entendu que les copules acides et alcools des esters pyréthrinoïdes ci-dessus peuvent exister sous toutes leurs formes stéréoisomères possibles.

**14)** Les compositions telles que définies à l'une des revendications 8 à 12, caractérisées en ce qu'elles renferment en outre un synergiste des pyréthrinoïdes.

**15)** Procédé de préparation des composés de formule (I) tels que définis à l'une quelconque des revendications 1 à 4, caractérisé en ce que l'on soumet un acide de formule (II) :

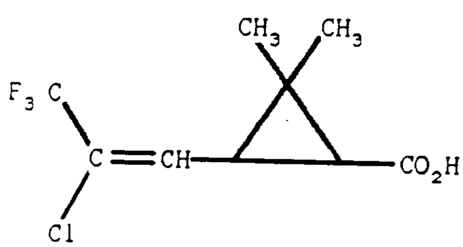

(II)

ou un dérivé fonctionnel de cet acide, avec un alcool de formule (III) :

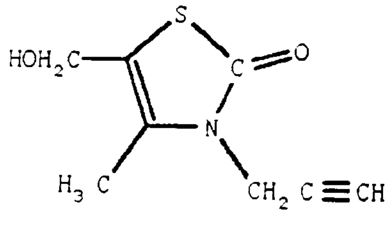

(III)

ou un dérivé fonctionnel de cet alcool, pour obtenir le composé de formule (I) correspondant.

Office européen des brevets

# RAPPORT DE RECHERCHE EUROPEENNE

Numero de la demande

EP 92 40 1873

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| X | EP-A-0 300 898 (ROUSSEL-UCLAF)<br>* page 11; revendications 1-5,8,16,21-29; exemple 7 *<br>--- | 1-15 | C07D277/34<br>A01N53/00 |
| A | FR-A-2 500 451 (ROUSSEL-UCLAF)<br>* revendications *<br>----- | 1,7-15 | |
| | | | DOMAINES TECHNIQUES RECHERCHES (Int. Cl.5 ) |
| | | | C07D |

Le présent rapport a été établi pour toutes les revendications

| Lieu de la recherche | Date d'achèvement de la recherche | Examinateur |
|---|---|---|
| LA HAYE | 01 OCTOBRE 1992 | HENRY J.C. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)